# EUROPEAN PATENT SPECIFICATION

(11) **EP 3 733 196 B1**
(45) Date of publication and mention of the grant of the patent: **30.08.2023**
(21) Application number: 18845399.7
(22) Date of filing: 21.12.2018
(51) Int. Cl.: A61K 38/17, C07K 14/47, A61P 29/00

(54) **PHARMACEUTICAL COMPOSITION COMPRISING AN ALTERED PEPTIDE LIGAND (APL) TYPE PEPTIDE**
PHARMAZEUTISCHE ZUSAMMENSETZUNG, DIE EIN PEPTID VOM TYP ALTERED PEPTIDE LIGAND (APL) ENTHÄLT
COMPOSITION PHARMACEUTIQUE COMPRENANT UN PEPTIDE DE TYPE ALTERED PEPTIDE LIGAND (APL)

(30) Priority: 29.12.2017 CU 20170176
(43) Date of publication of application: 04.11.2020
(73) Proprietor: Centro De Ingenieria Genetica Y Biotecnologia, La Habana 11600 (CU)
(72) Inventor: DOMÍNGUEZ HORTA, María del Carmen, Cubanacán, Playa, La Habana 11600 (CU); LOPEZ ABAD, Cruz Matilde, Playa La Habana 11600 (CU); GONZÁLEZ LÓPEZ, Luis Javier, Playa La Habana 11600 (CU); BESADA PÉREZ, Vladimir Armando, Playa La Habana 11600 (CU)
(74) Representative: V.O.
(86) International application number: PCT/CU2018/050007
(87) International publication number: WO 2019/129315

(56) References cited:
- EP-A1- 2 371 847
- EP-A1- 2 374 468
- WO-A2-2007/124082
- CABRALES-RICO ANIA ET AL: "Development and validation of a bioanalytical method based on LC-MS/MS analysis for the quantitation of CIGB-814 peptide in plasma from Rheumatoid Arthritis patients", JOURNAL OF PHARMACEUTICAL AND BIOMEDICAL ANALYSIS, NEW YORK, NY, US, vol. 143, 1 June 2017 (2017-06-01), pages 130-140, XP085108197, ISSN: 0731-7085, DOI: 10.1016/J.JPBA.2017.05.030
- BARBERÁ ARIANA ET AL: "APL1, an altered peptide ligand derived from human heat-shock protein 60, increases the frequency of Tregs and its suppressive capacity against antigen responding effector CD4 + T cells from rheumatoid arthritis patients", CELL STRESS AND CHAPERONES, ALLEN PRESS ONLINE PUBLISHING, EDINBURGH, GB, vol. 21, no. 4, 30 May 2016 (2016-05-30), pages 735-744, XP035981934, ISSN: 1355-8145, DOI: 10.1007/S12192-016-0698-0 [retrieved on 2016-05-30]
- SATOSHI OHTAKE ET AL: "Interactions of formulation excipients with proteins in solution and in the dried state", ADVANCED DRUG DELIVERY REVIEWS, vol. 63, no. 13, 1 July 2011 (2011-07-01), pages 1053-1073, XP055580451, AMSTERDAM, NL ISSN: 0169-409X, DOI: 10.1016/j.addr.2011.06.011

## Description

### Field of technique

Present invention relates with a branch of medicine, in particular to a pharmaceutical composition comprising a modified peptide type APL (Altered Peptide Ligand, abbreviated APL). Administration of this composition to patients decreases events associated with protein citrullination. The composition is useful for treatment of inflammatory diseases such as rheumatoid arthritis (RA), ankylosing spondylitis (AS), juvenile idiopathic arthritis (JIA), liver fibrosis and Alzheimer's disease.

### State of previous technique

Citrullination is a post-translational modification of proteins that transforms the guanidinium group (positive charge) from arginine to a citrullinated (neutral) ureido group. Enzymes peptidil-arginine deiminases (PAD) catalyzing this transformation (Bicker K. L., Thompson P. R. 2013, Biopolymers, 99: 155-163). In RA, exposure to environmental agents that trigger the disease and NETosis (NETs) of neutrophils represent the main sources of citrullinated autoantigens. NETosis is a programmed cell death in which the rupture of the plasma membrane occurs, associated with changes in the nuclear morphology of neutrophils. Liberation of the cytoplasmic content of neutrophils in the extracellular space, form a matrix of chromatin enriched with antimicrobial protein granules and enzymes, so-called extracellular traps of neutrophils. In this process enzymes PADs and citrullinated histones H3 are released, which become potent autoantigens (Konig MF, Andrade F. 2016. Front Immunol 7: 461), and increase the inflammatory response of synovial fibroblasts that invade to cartilage. On the other hand, environmental agents that trigger RA cause apoptosis and death due to necrosis of cells that form the extracellular matrix. Necrotic cells release their cytoplasmic content into the extracellular space. During this process, PAD enzymes are also released, which are activated due to levels of calcium in the extracellular space and cause citrullination to self-proteins.

NETosis of neutrophils is also associated with the pathogenesis of AS and JIA (Giaglis S et al., 2016. Front, Pediatr, 4:97). On the other hand, NETosis can exacerbate neuro-inflammatory processes, promotion of vascular damage in the parenchyma from patients with Alzheimer's disease (Pietronigro EC et al., 2017. Front Immunol 8: 211).

In addition, NETosis is a biological process that contributes to the development of fibrosis in various tissues such as lung and liver, associated with citrullination of vimentin (Branzk N, Papayannopoulos V. Seminars in Immunopathology, 2013; 35 (4): 513-530).

In particular, several proteins that are targets of citrullination have been described in RA, among which are fibrinogen, keratin, fibronectin, vimentin, type II collagen and α-enolase (McInnes IB, Schett G. 2011 N Engl J Med. 365 (23): 2205-2219). On the other hand, it has been found that citrullination of vimentin contributes significantly to the pathogenesis of AS, JIA, hepatic and pulmonary fibrosis, and Alzheimer's disease (S. Gudmann et al., 2015. Autoimmunity 48: 73- 79).

During RA progressing, B cells proliferate and differentiate into cells secreting anti-citrullinated protein antibodies (ACPAs), after interaction with helper T cells activated by citrullinated peptides. ACPAs are found in 50% from patients, approximately one year before of RA debut. Studies in animal models suggest that migration of these autoantibodies, from extra-articular sites to the synovium, trigger joint inflammation. In addition, presence of ACPAs in the synovium is a risk factor for this disease, because these autoantibodies recognize citrullinated vimentin molecules, and induce the differentiation of mononuclear cells into osteoclasts, which promote bone resorption and destruction ( Martin-Mola E. et al 2016. Rheumatol Int. 36 (8): 1043-1063).

A factor that contributes markedly to RA pathogenesis is the activation of neutrophils by immune complexes deposited in synovial fluid or on cartilage surface (Rollet-Labelle E. et al., 2013. J Inflamm (Lond). 1): 27). Neutrophils induced synthesis and release cytotoxic products (prostaglandins, proteases and oxygen free radicals), which severely damage the joint. Neutrophils orchestrating inflammatory process through four mechanisms: (1) secretion of cytokines and chemokines, including IL-23, IL-12, RANKL (Activator Receptor for Nuclear Factor-κB Ligand) and IL-18, which caused activation of osteoclasts and B lymphocytes; (2) increased expression of molecules from major histocompatibility class II complex; (3) cell-cell interactions that cause the activation of other cell types that destroy to joint; and (4) release of proteases that activate or inactivate cytokines and chemokines involved in joint damage (Wright H. L et al. (2014. Nat Rev Rheumatol. 10 (10): 593-601).

Neutrophils have a half-life, short, approximately between 30 minutes and 8 hours. Proteins S100A8/9 represent 40% of proteins in the cytosol from these cells; this complex induced programmed cell death of neutrophils (M Atallah, et al., 2012. PLoS ONE 7: 2 (e29333). S100A8 and S100A9 usually are in a complex known as calprotectin, both S100A8 and S100A9 and calprotectin, cause chemotaxis of neutrophils, in very low concentrations (nanomolar range). Calprotectin (S100A8/9) is found in high concentrations in local sites of inflammation or in the serum of patients with inflammatory diseases such as RA, fibrosis and Crohn's disease (Cerezo, L. et al. 2011. Arthritis Res. Ther. 13 (4): R122).

Cohen and Monsonego in the patent application No. WO2009090650 claims use of a peptide derived from HSP60, in combination with the β-amyloid protein, for the treatment of Alzheimer's disease. Said peptide comprises the sequence of amino acids 458-474 from HSP60. Other authors revealed the use of small peptides that also prevent the aggregation of β-amyloid protein for treatment of Alzheimer's disease (P Sundaram et al., Patents on Potential Drugs to Treat Alzheimer's Disease: Special Emphasis on Small Peptides, 2014. 9: 71-75). Patent application No. PCT / IL2003 / 000132 claims the use of peptide p277, derived from HSP60, which is able to modulate activity of T cells, through the TLR type 2 , for treatment of various diseases chronic inflammations, among which cites pulmonary fibrosis. In the patent application WO2006032216 is claimed the APL type peptide identified in the Sequence Listing as Seq ID No. 1; and its use in the treatment of RA. On the other hand, patent EP2374468 discloses the use of said peptide in the treatment of Crohn's disease, ulcerative colitis and type I diabetes mellitus.

Currently, there are no effective therapies that cause the decrease of ACPA levels, NETosis and the biological process of protein citrullination. There is also no effective cure for autoimmune arthritis such as RA, JIA and AS, or the other diseases that are also characterized by the citrullination of their self-proteins, such as Alzheimer's disease and liver and lung fibrosis. Therefore, it is still interesting to obtain drugs that offer therapeutic advantages with respect to the drugs that exist in the treatment of this type of diseases.

### Explanation of invention

The present invention resolves aforementioned problem, since it provides a pharmaceutical composition comprising APL type peptide identified as SEQ ID No. 1, sodium acetate buffer at pH 3.9-4.7; and at least one stabilizing sugar selected:
sucrose or trehalose. Peptide identified as Seq ID No. 1 is derived from a region of human HSP60, comprised between amino acids 83 to 109. APLs type peptides are similar to immunogenic peptide analogs, but with one or more substitutions in the essential contact positions with T cell receptor, or with the major histocompatibility complex class II , which modifies the cascade of events necessary for activation of T cells. Concentrations of this peptide in the pharmaceutical composition at present invention allow an effective immunological response in the host.

The peptide of Seq ID No. 1 is produced by chemical synthesis. Level and quality of the immune response that it induces can be evaluated in experiments such as those described in the present invention. In an example of this invention the peptide is in a concentration between 0.5 mg / mL and 10 mg / mL in pharmaceutical composition, Sodium acetate buffer has a concentration between 30 mM and 70 mM. On the other hand, in an example of this invention stabilizing sugar is in the range between 10 mg/ mL and 40 mg/mL.

Pharmaceutical composition of this invention is characterized by its high stability and safety. This was verified in patients during examination. Pharmaceutical composition was well tolerated. No serious adverse events were identified in any patient, biochemical or hematological parameters were not modified. These results were unexpected, taking into consideration that pH of formulation is around 4.0; or distant from physiologic pH. In similar formulations of other compounds, where the pH is far from the physiological pH, adverse events at the injection site have been observed, such as irritability and pain. In an embodiment of this invention, the composition that is administered to patients is liquid or lyophilisate.

In the present invention is revealed the capacity of the pharmaceutical formulation comprising of peptide identified as Seq ID No. 1, excipient sucrose or trehalose, and sodium acetate buffer at pH between 3.9 and 4.7, for reducing percentage of neutrophils, NETosis and levels of ACPAs. These pathogenic events are involved in the development of RA, JIA, AS, Alzheimer's disease and fibrosis. Pharmaceutical composition described above significantly decreased the number of neutrophils in ex vivo assays carried out with cells isolated from patients with these diseases.

Surprisingly, this pharmaceutical preparation also significantly decreased cyclic citrullinated peptide anti-CCP (anti-CCP) levels in a group of patients with RA who were treated with this preparation. In addition, the peptide decreased the levels of S100A9 and S100A8 proteins in ex vivo assays, performed with mononuclear cells isolated from patients with RA.

Therefore, another aspect of this invention is the use of said pharmaceutical composition for manufacturing a medicament for treatment of an inflammatory disease related to an increase in neutrophils or citrullination of proteins. The use of antigen-specific strategies that regulate the exacerbated immune response, with consequent decrease in citrullination process characteristic of these diseases, represents a very novel therapeutic approach. In an embodiment of the invention, the composition is used to make a medicament for an inflammatory disease that is selected from the group consisting of RA, JIA, AS, Alzheimer's disease, and hepatic and pulmonary fibrosis.

It had been suggested that peptide identified as Seq ID No. 1 could be useful for treatment of RA (Dominguez MC et al., 2011. Autoimmunity 44 (6): 471-482; Barberá A. et al., 2016. Cell Stress and Chaperones 21: 735-744). However, there is no evidence to suggest that a particular formulation of this peptide can be used for treatment of inflammatory diseases where citrullination of protein is increased and antibodies against citrullinated proteins are present.

In another aspect, the invention provides a method for the treatment of an inflammatory disease related to an increase of neutrophils or protein citrullination. This method comprises administering to a needy subject thereof a therapeutically effective amount of the pharmaceutical composition of the invention. In anembodiment of the invention, the inflammatory disease is selected from group consisting of RA, JIA, AD, Alzheimer's disease, and hepatic and pulmonary fibrosis.

The effective amount of composition is that which, when is administered, results in a decrease in the levels of anti-CCP antibodies, NETosis antibodies and the consequent protein citrullination in the patients. In the course of treatment, the amount of peptide administered may be modified, according to certain factors such as age, sex, general health, as well as the level of immune response in general.

In another embodiment of the invention, as part of the method, anti-inflammatory drugs or cytokine antagonists are also administered to the patients. In a particular embodiment, in the method of treating inflammatory diseases, the pharmaceutical composition comprising the APL-type peptide is liquid or lyophilized, which is dissolved to be administered.

In one embodiment, the pharmaceutical composition is administrable parenterally or mucosally. In a preferred embodiment, the pharmaceutical composition of the peptide is administered subcutaneously. In another embodiment, the pharmaceutical composition is administered orally.

### Brief description of the figures

**Figure 1****.** Chromatographic profile of APL-type peptide identified as Seq ID No. 1 dissolved in 50 mM sodium phosphate buffer; pH 7.4. The profile was obtained by reverse phase chromatography (RP-HPLC), (A) time 0, (B) 15 days of storage at 37 ° C.
**Figure 2****.** Evaluation of the solubility of the peptide identified as Seq ID No. 1 in several excipients, determined by RP-HPLC.
**Figure 3****.** Molecular exclusion chromatography - HPLC of the peptide of Seq ID No. 1 dissolved in water (A), in phosphate buffered saline (PBS) (B) and in sucrose (C).
**Figure 4****.** Effect of pH on concentration of peptide identified as Seq ID No. 1, in samples formulated and stored at 4 ° C (A) and at 37 ° C (B).
**Figure 5****.** Effect of pH on the purity of the peptide identified as Seq ID No. 1, in samples formulated and stored at 37 ° C.
**Figure 6****.** Effect of pharmaceutical composition of peptide on cell viability of neutrophils obtained from peripheral blood from patients with AS. Cells cultured without peptide stimulation are negative control.
**Figure 7****.** Effect of administration of a pharmaceutical composition of peptide on the concentration of anti-CCP in patients with RA treated with it. The asterisks indicate statistically significant differences between time 0 (without treatment) and the three times evaluated.

### Detailed description examples

### Example 1. Obtaining an APL-type peptide formulation for use in humans.

APL type peptide was obtained by chemical synthesis. It was observed that by dissolving peptide identified as Seq ID No. 1 in 50 mM sodium phosphate buffer; pH 7.4, this is progressively destabilized. To study the causes of such effect, peptide was dissolved in that buffer and stored at 37 ° C for 15 days. Subsequently, several aliquots were analyzed periodically by RP-HPLC. Presence of contaminating species was verified, these were purified and analyzed by mass spectrometry, and several fractions were identified that could correspond to degradations.

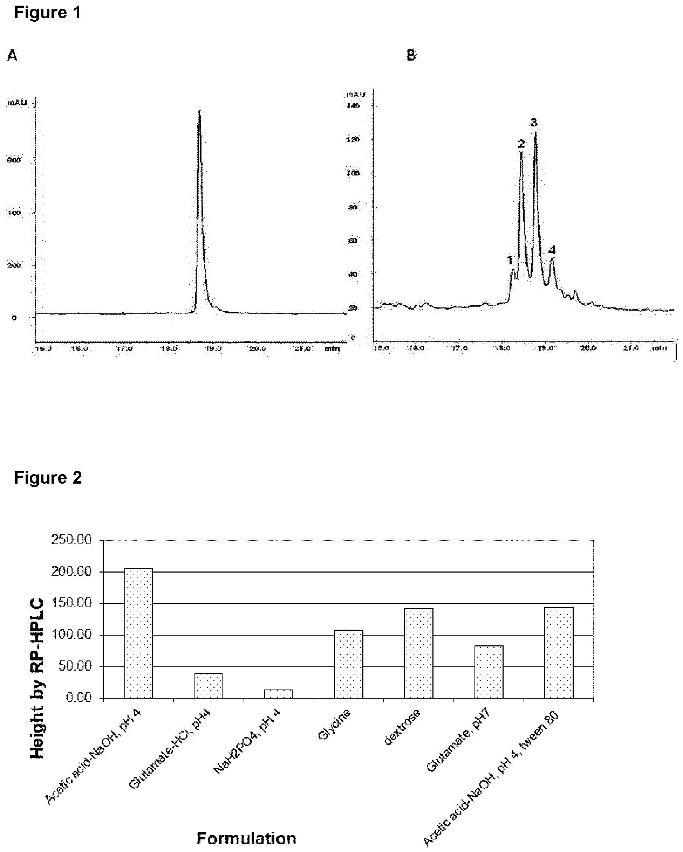

In order to accelerate the degradation, peptide was incubated under stress conditions that allow the possible degraded species of the peptide to be obtained fast and in high proportions. Fractions collected after RP-HPLC were characterized by mass spectrometry. Chromatographic profile of the intact peptide at zero time is shown in Figure 1A. Figure 1B shows profile of the same peptide after being incubated for 15 days at 37 ° C, under the aforementioned conditions. After analysis by mass spectrometry of the fractions observed in Figure 1B, it was determined that fractions 1, 2 and 4 correspond to deamidated variants of the peptide, while fraction 3 corresponds to the intact peptide.

Peptide has four asparagine, which explains the deamidated species obtained, which affect its stability when dissolved in 50 mM sodium phosphate buffer; pH 7.4. Additionally, the peptide is very poorly soluble in this buffer, which makes it difficult to prepare a pharmaceutical formulation that can be administered to patients at the effective dose. Taking these results, pre-formulation studies were performed, to obtain a stable pharmaceutical product comprising the peptide. Different ionic species, pH and excipients were evaluated, to achieve the total solubility of the peptide, and a high chemical stability. Solubility of peptide was evaluated with different excipients, which included different ionic salts, sugars, polyols, amino acids and detergents.

First stage of the formulation development included the evaluation of buffer species influence and pH on chemical and physical stability of the peptide. This is poorly soluble in sodium phosphate buffer, and very poorly soluble in other ionic species, such as sodium citrate, sodium glutamate and Tris-HCl. Figure 2 shows solubility of the peptide in some of the excipients evaluated. Sodium acetate buffer, prepared with acetic acid and sodium hydroxide, was the one that best solubilized the peptide, when evaluating buffer concentrations between 10 and 70 mM.

On the other hand, Table 1 summarizes the results of solubility evaluation of peptide, formulated at concentrations between 1 and 10 mg / mL, with different types of pharmaceutically acceptable excipients. It is noted that the highest solubility of the peptide was obtained in solutions of sucrose or trehalose, followed by the sodium acetate buffer. Other excipients did not stabilize the peptide under study in the same way.

**Table 1. Effect of evaluated excipients on peptide solubility**

| **Excipient** | **Concentration** | **Solubility** |
|---|---|---|
| Sugar: Sucrose, Trehalose | 10 - 40 mg/mL | +++++a |
| Azúcar: Dextrose | 30 - 50 mg/mL | +++ |
| Polyols: Mannitol, Sorbitol | 5-15 mg/mL | +++ |
| Polymers: Dextran,β-cyclodextrin, | 10 - 20 mg/mL | +++ |
| Detergents: Tween 80, Tween 20 | 0,005 - 0,05% | ++ |
| Ionic species: Sodium hydrogen phosphate and sodium dihydrogen phosphate | 10 mM - 50 mM | + |
| Buffer Sodium citrate | 10 mM - 50 mM | + |
| Buffer sodium acetate | 10 mM - 70 mM | ++++ |
| Sodium glutamate | 10 mM - 50 mM | ++ |
| Buffer Tris-HCl | 10 mM - 50 mM | ++ |
| Sodium chloride | 5-15 mg/mL | + |
| Amino Acid: Glycine | 10 - 20 mg/mL | ++ |

| | | |
|---|---|---|
| a: +++++: Maximum solubility, +: very low solubility | | |

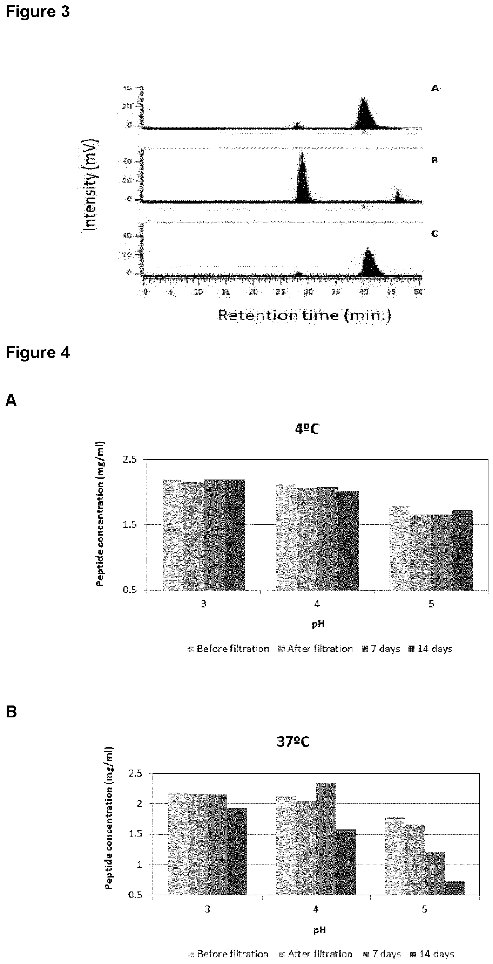

Analysis of peptide dissolved in water, in sucrose (20 mg / mL) and in PBS was also performed by HPLC, which showed the influence of sugar on the physical stability of peptide. In PBS the peptide eluted in a shorter retention time, compared to the sample dissolved in water and in sucrose solution. This indicates that PBS can favor the interactions between monomers of peptide, therefore in chromatography elutes as aggregated. However, sucrose inhibits the interaction between molecules of peptide, and this elutes, in a majority as a species with a longer retention time, which may correspond to the monomer of peptide. This result, which is show in Figure 3, corroborates best solubility profile of peptide obtained with sucrose solution. Peptide dissolved in water, which is used as a control, also achieves a good solubility. However, it does not constitute a buffer species, which is necessary for formulation of the peptide its use as drug.

From these results, for further studies peptide was formulated at 2.5 mg / mL, in 50 mM sodium acetate buffer, and influence of pH on chemical and physical stability of peptide in the formulation was analyzed. Three pH values were analyzed: 3.0; 4.0 and 5.0. Samples were filtered and stored at 4 °C or at 37 °C, for 15 days. Evaluation of stability and concentration of peptide was carried out by RP-HPLC. Results of concentration of peptide stored at 4 ° C or at 37 ° C, respectively, are shown in Figures 4A and 4B, respectively. Peptide does not dissolve completely at pH 5.0, consequently concentration are lower than those obtained at pH 3.0 or pH 4.0. At 37 °C, precipitation process is accelerated. Over time, a decrease in the concentration of the peptide is observed in the three pHs studied, this effect being greater in the following order: pH 5.0 >pH 4.0 > pH 3.0. Under these conditions analyzed, physical stability of peptide was greater at pH 3.0.

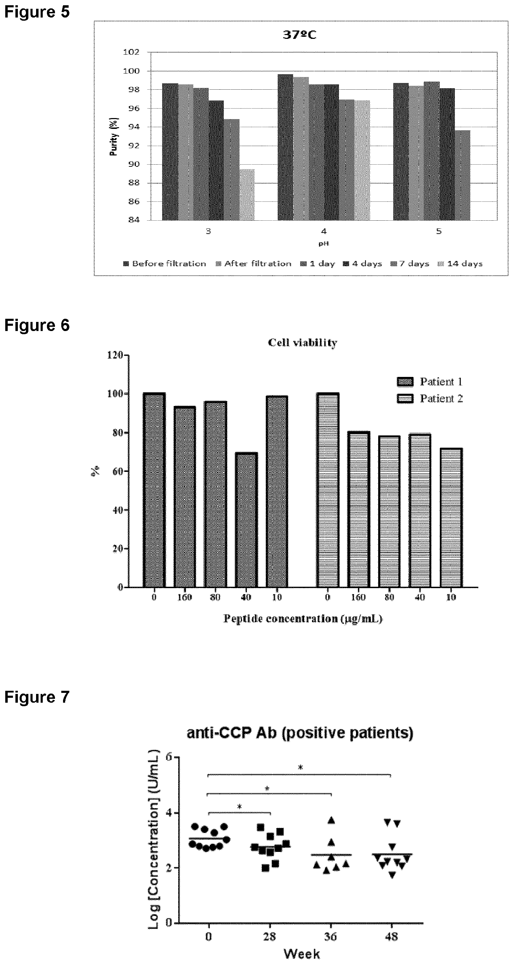

Chemical integrity of peptide (expressed as % purity in RP-HPLC) was greater than 95%, for the samples formulated in three pH values, when these were stored at 4°C, for 15 days. However, when the samples were incubated at 37 ° C, a tendency to decrease in purity was observed, this being larger for samples formulated at pH 3.0. These results are shown in Figure 5, which indicate that pH 3.0 favors the chemical degradation of peptide.

In correspondence with these results, it was concluded that the optimal formulation is that where the peptide is in sodium acetate buffer, in a range between 30 mM and 70 mM, at pH around 4.0. In addition, according with these results of solubility study (Table 1), it was decided to use as stabilizing sugar sucrose or trehalose, at a concentration between 10 and 40 mg / mL, to prepare a lyophilized composition of APL-type peptide under study. These stabilizing sugars were essential for the complete solubility of peptide, and these sugars were also important for storage in lyophilized state, since contributed stability to peptide.

In the composition described, no molecular species corresponding to degradations of peptide were detected. This pharmaceutical composition proved to be a consistent product throughout the manufacture of several batches. This showed a real stability of 24 months stored at 4°C.

### Example 2. Decrease of neutrophils in ex vivo assays performed with cells from treated patients.

As mentioned above, neutrophils have a crucial role in the progressing and chronicity of several inflammatory diseases. Therefore, the effect of pharmaceutical composition of APL-type peptide identified as Seq ID No. 1, which was selected in Example 1, on viability of neutrophils from patients were studied.

Blood from patients was diluted 1: 2 with the 3% dextran T500 solution, mixed by repeated inversion of the tubes, and incubated for 18-20 min at room temperature for pelleting of erythrocytes. Upper stratum, rich in leukocytes, was extracted and transferred to 50 mL tubes. Tubes were centrifuged at 500xg, for 10 min at 4 ° C. Cell pellet was resuspended in 10 mL of ion-free PBS. Five milliliters of this cell suspension was transferred to 15 mL centrifuge tubes, which contained 3 mL of Ficoll-Paque ^{™} PLUS (Amersham Biosciences AB, Sweden). These tubes were centrifuged at 400xg, for 40 min, at room temperature. After centrifugation, four layers were observed, corresponding to plasma plus PBS, peripheral blood mononuclear cells (PBMC), Ficoll-Paque solution, and layer of granulocytes plus erythrocytes. The layer corresponding to granulocytes was extracted. The remaining erythrocytes were lysed, by the addition of sterile distilled water. It was centrifuged at 500xg for 5 min at 4 ° C. Neutrophils were resuspended in RPMI 1640 culture medium, containing 10% fetal bovine serum and supplemented with gentamicin (100 U / mL) and L-glutamine (2 mM) (Gibco BRL, USA).

Neutrophils were plated (1×10⁶ cells / well) in 96-well plates (Costar, USA), in a final volume of 100 µL, and stimulated (in triplicate) with the composition selected in Example 1, at the following concentrations of peptide: 10, 40, 80 and 160 µg/mL. Stimulation was carried out for 24 h. Cells not stimulated with said composition were used as control of basal cell growth. Effect of peptide formulation on the viability of the cells was determined with the assay of 3- [4,5-dimethylthiazol-2-yl] -2,5 diphenyl tetrazolium bromide (abbreviated MTT, Sigma, USA), following the protocol described by manufacturer. After 24 h of culture, 20 µL / well of MTT, prepared at a concentration of 5 mg/mL in culture medium, was added. Plates were then incubated for 4 h in the same culture conditions. At the end of this time, 100 µL of dimethylsulfoxide was added, and content of wells was homogenized. Average of optical densities of unstimulated cells was considered 100% of cell viability. From this value, the viability of cultured cells was calculated with peptide formulation, according to the average optical densities in each case.

As seen in Figure 6, a decrease in cell viability of neutrophils from patients with AS was very marked, with regarding to cells without peptide stimulation. Formulated peptide decreased cell viability by up to 35%, when compared to cells without peptide stimulation. Results very similar to these were found in the experiments that were performed with neutrophils isolated from patients with other inflammatory diseases (Table 2). These results support the use of the selected composition of this peptide in the treatment of said diseases, since it decreases a cell population involved in the pathogenesis of these pathologies.

**Table 2. Effect of pharmaceutical composition of APL-type peptide on cell viability of neutrophils from patients with inflammatory diseases**

| **Disease** | **No. of patients** | **% inhibition** |
|---|---|---|
| AR * | 3 | 35 |
| JIA** | 2 | 35 |
| Crohn's disease | 2 | 30 |
| Pulmonary fibrosis | 2 | 25 |
| Alzheimer's disease | 2 | 30 |

| | | |
|---|---|---|
| * Patients who presented severe activity. ** Patients from polyarticular subtype with severe activity | | |

However, when pharmaceutical formulations of peptide prepared with other excipients were analyzed, it was found that effect of decreasing the cell viability of the neutrophils was markedly reduced. This is observed in Table 3. These cells were isolated from peripheral blood of patients with inflammatory diseases characterized by an increase in protein citrullination, at the rate of one patient for each disease. For isolation of these cells under study, the protocol described above was followed.

**Table 3. Effect of the excipients present in the formulation of APL-type peptide on cell viability of neutrophils from patients with inflammatory diseases**

| | | % **inhibition in cell viability of neutrophils from patients** | | | | | |
|---|---|---|---|---|---|---|---|
| **Excipient** | **Concentration** | **RA** | **AS** | **JIA** | **Crohn^{a}** | **Fibrosis^{b}** | **Alzheimer^{c}** |
| Sucrose or Trehalose in Sodium Acetate * | 10- 40 mg/mL | 35 | 35 | 35 | 30 | 30 | 35 |
| Ionic species: Sodium hydrogen phosphate and sodium dihydrogen phosphate | 10 mM - 50 mM | 5 | 5 | 0 | 0 | 0 | 5 |
| Citric acid-Sodium Citrate | 10 mM - 50 mM | 0 | 0 | 0 | 0 | 0 | 0 |
| Sodium glutamate | 10 mM - 50 mM | 20 | 15 | 15 | 15 | 20 | 20 |
| Tris-HCl | 10 mM - 50 mM | 20 | 15 | 20 | 20 | 15 | 20 |
| Sodium chloride | 5 - 15 mg/mL | 5 | 5 | 5 | 5 | 5 | 0 |
| Amino Acids: Glycine | 10 - 20 mg/mL | 10 | 10 | 5 | 5 | 10 | 10 |

| | | | | | | | |
|---|---|---|---|---|---|---|---|
| a: Crohn's disease, b: liver fibrosis, c: Alzheimer's disease. *: Pharmaceutical composition selected in Example 1, used as control of the experiment. | | | | | | | |

These results confirm that formulation of the peptide in sodium acetate buffer (30-70 mM), at pH 3.9-7.7; and sucrose or trehalose (10-40 mg / mL) is useful to induce a decrease in cell viability of neutrophils isolated from patients with RA, JIA, AS, fibrosis and Alzheimer's disease.

### Example 3. Decrease of S100A8 and S100A9 and NETosis proteins in ex vivo assays with cells isolated from patients with RA.

The samples derived from three female patients with RA, with 5 years of disease evolution, and an age between 50 to 55 years. At the time of blood collection, patients had a moderate activity of disease, according to DAS28 (Disease Activity Score) clinical index, which is index of disease activity, known to experts in this branch of medicine. (PL Van Riel and J. Fransen, 2005. Arthritis Res Ther 7: 189-190).

Blood from patients was diluted 1: 2 with a phosphate buffer solution. Isolation of mononuclear cells was performed, with a Ficoll gradient. Cells were re-suspended in RPMI 1640 medium containing 10% fetal bovine serum. Ten million cells were cultured at 37 ° C in a humid atmosphere of 5% CO2, with peptide formulated (at 40 µg/mL) as proceeding in Example 1, for 24 hours or 5 days. In addition, unstimulated cells were cultured with peptide, which constituted controls of experiments.

After the culture, cells were treated with a saline solution rich in detergents, proteases and with oxygen isotope 18, to cause the rupture of plasma membrane and marking of proteins. Samples were analyzed in an LC / MS / MS mass spectrometer, performing 18 runs for each sample.

Subsequently, biological interpretation of results was performed, for which proteins that changed significantly in each cell cultures were determined, both at 24 hours and at 5 days of incubation with the pharmaceutical composition comprising peptide. All proteins were characterized in terms of their biological function, their possible post-translational modifications and their relationship with diseases. Using the total list of proteins that changed significantly, enrichment analyzes were performed by metabolic pathways, by molecular processes, by interaction networks and by diseases. This procedure was also applied to samples of each patient independently, comparing results to the two study times, and to the global analysis.

On the other hand, those identified proteins were selected that had same behavior in at least two of patients studied, or in the samples of both study times in a single patient. With this new list of proteins, text mining was performed to identify those that were directly related to the disease. The proteins that were identified with this strategy were studied in terms of their functions, and their quality as a biomarker for disease.

The enrichment analysis was carried out by maps, metabolic routes and biological processes, comparing all experiments among each other (each patient, cells evaluated at 24 hours and at 5 days). Among maps or biological routes most represented are NETosis and those related to response to damage in deoxyribonucleic acid. When analyzing proteins identified in the three patients, S100A8 and S100A9 were found as relevant proteins.

These proteins decreased in the cells from three patients, when these were cultured with pharmaceutical composition comprising the peptide, both for 24 hours and for 5 days, in comparison with cells without peptide stimulation.

Said pharmaceutical composition also markedly decreased NETosis. S100A8 and S100A9 proteins are present in neutrophils, where constitute 40% of cytosol proteins, but are also present in monocytes. However, NETosis is a process particularly associated with neutrophils. This last point can be explained by the fact that it is probable that the ring of mononuclear cells isolated from the patients was contaminated with neutrophils. This is a very likely fact, considering that the three patients were found to have moderate activity of the disease, where the number of neutrophils is increased.

On the other hand, these results suggest that referred pharmaceutical composition, comprising APL-type peptide and selected excipients, may contribute to decrease citrullination of proteins characteristic of several diseases such as autoimmune arthritis, fibrosis and Alzheimer's disease. Citrullination is a process that favors development of these pathologies, and precisely the products that are released to extracellular environment during NETosis are precursors of citrullination.

### Example 4. Decrease of anti-CCP antibody levels in patients with RA treated with composition comprising the APL-type peptide.

Blood samples analyzed derived from patients with RA, included in a phase I clinical trial. Patients were inoculated with composition comprising APL type peptide identified as Seq. ID No. 1, which was described in Example 1. At the time of inclusion, patients had moderate activity of disease, according to DAS28 clinical index.

Clinical trial was structured in three dose levels of peptide: 1 mg, 2.5 mg and 5 mg. Patients were treated with pharmaceutical composition of peptide for six months. They received nine administrations of said formulation, subcutaneously, divided into a weekly dose during the first month, and a monthly dose for five months.

Patients were followed up for detection of adverse events. Pharmaceutical composition of APL-type peptide administered was well tolerated by all patients. No serious adverse events were identified in any patient, and no biochemical or hematological parameters were modified. These results were surprising, taking into consideration that the pH of the formulation is around 4.0; it is far from the physiological pH. In previous studies by other authors, damage has been reported locally at the site of administration, when the pH of the formulation administered was far from the physiological pH.

Additionally, concentration of anti-CCP antibodies was quantified in the plasma of the patients, by a commercially ELISA-type assay. This ELISA allows the determination of antibodies against four antigens: Vimentin, Collagen type II, Fibrinogen and α-enolase. Results were expressed in pg/mL, and analyzed with the GraphPad Prism statistical program. T-test was applied for comparison of times evaluated with respect to time zero.

Surprisingly, as seen in Figure 7, treatment with selected APL-type peptide formulation induced a significant decrease in concentration of anti-CCP antibodies, in the plasma from treated patients, with respect to time zero (p<0,05). Patient samples corresponded to 13, 25 and 48 weeks of treatment. These results demonstrated a therapeutic effect of this formulation, since anti-CCP antibodies contribute to pathogenesis of RA, and other inflammatory diseases such as JIA, AS, Alzheimer's disease, and hepatic and pulmonary fibrosis. On the other hand, results indicate that said pharmaceutical composition, which comprises APL type peptide, can contribute to decrease citrulination process of self-proteins, characteristic of the mentioned diseases.

## Claims

1. A pharmaceutical composition comprising APL type peptide identified as SEQ ID No. 1, sodium acetate buffer at pH 3.9-4.7; and at least one stabilizing sugar selected from sucrose and trehalose.

2. Composition according to claim 1 wherein peptide is in a concentration between 0.5 mg/mL and 10 mg / mL.

3. Composition according to claim 1 wherein sodium acetate buffer has a concentration between 30 mM and 70 mM.

4. Composition according to claim 1 wherein stabilizing sugar is in the range between 10 mg / mL and 40 mg/mL.

5. Composition according to any one of claims 1 to 4, which is in liquid form or is the product of resuspending a lyophilizate.

6. Composition according to any one of claims 1 to 5 for use as a medicament.

7. Composition for use according to claim 6, wherein said composition is for use in the treatment of an inflammatory disease related to an increase in neutrophils or protein citrullination; and wherein the inflammatory disease is selected from a group consisting of rheumatoid arthritis (RA), juvenile idiopathic arthritis (JIA), ankylosing spondylitis (AS), Alzheimer's disease, and hepatic or pulmonary fibrosis.

## Patentansprüche

1. Pharmazeutische Zusammensetzung, umfassend ein APL-Typ Peptid, identifiziert als SEQ ID Nr. 1, Natriumacetatpuffer mit pH 3,9-4,7; und wenigstens einen stabilisierenden Zucker, ausgewählt aus Saccharose und Trehalose.

2. Zusammensetzung nach Anspruch 1, wobei das Peptid in einer Konzentration zwischen 0,5 mg/mL und 10 mg/mL ist.

3. Zusammensetzung nach Anspruch 1, wobei der Natriumacetatpuffer eine Konzentration zwischen 30 mM und 70 mM hat.

4. Zusammensetzung nach Anspruch 1, wobei der stabilisierende Zucker in dem Bereich zwischen 10 mg/mL und 40 mg/mL ist.

5. Zusammensetzung nach einem der Ansprüche 1 bis 4, die in flüssiger Form ist oder das Produkt der Resuspendierung eines Lyophilisats ist.

6. Zusammensetzung nach einem der Ansprüche 1 bis 5 zur Verwendung als ein Arzneimittel.

7. Zusammensetzung zur Verwendung nach Anspruch 6, wobei die Zusammensetzung zur Verwendung bei der Behandlung einer entzündlichen Erkrankung ist, die mit einem Anstieg an Neutrophilen oder Protein-Citrullinierung zusammenhängt, und wobei die entzündliche Erkrankung ausgewählt ist aus einer Gruppe, bestehend aus rheumatoider Arthritis (RA), juveniler idiopathischer Arthritis (JIA), ankylosierender Spondylitis (AS), Alzheimer-Krankheit, und Leber- oder Lungenfibrose.

## Revendications

1. Composition pharmaceutique comprenant un peptide de type APL, identifié par la Séquence N° 1, du tampon acétate de sodium à un pH de 3,9 à 4,7, et au moins un sucre stabilisant, choisi parmi du saccharose et du tréhalose.

2. Composition conforme à la revendication 1, dans laquelle le peptide se trouve en une concentration valant entre 0,5 mg/mL et 10 mg/mL.

3. Composition conforme à la revendication 1, dans laquelle le tampon acétate de sodium présente une concentration valant entre 30 mM et 70 mM.

4. Composition conforme à la revendication 1, dans laquelle le sucre stabilisant se trouve en une concentration située dans l'intervalle entre 10 mg/mL et 40 mg/mL.

5. Composition conforme à l'une des revendications 1 à 4, qui se présente sous la forme d'un liquide ou qui est le produit de la remise en suspension d'un lyophilisat.

6. Composition conforme à l'une des revendications 1 à 5, pour utilisation en tant que médicament.

7. Composition pour utilisation, conforme à la revendication 6, laquelle composition est destinée à être utilisée dans le traitement d'une maladie inflammatoire en relation avec une augmentation des neutrophiles ou avec la citrullination des protéines, laquelle maladie inflammatoire est choisie dans l'ensemble constitué par les suivantes : polyarthrite rhumatoïde (PAR), arthrite juvénile idiopathique (AJI), spondylarthrite ankylosante (SA), maladie d'Alzheimer, et fibrose hépatique ou pulmonaire.
